# EUROPÄISCHE PATENTSCHRIFT

(11) **EP 1 756 026 B1**
(45) Veröffentlichungstag und Bekanntmachung des Hinweises auf die Patenterteilung: **18.03.2009**
(21) Anmeldenummer: 05757295.0
(22) Anmeldetag: 15.06.2005
(51) Int. Cl.: C07C 17/02, C07C 19/045

(54) **VERFAHREN UND VORRICHTUNG ZUR HERSTELLUNG VON 1,2-DICHLORETHAN MITTELS DIREKTCHLORIERUNG**
METHOD AND DEVICE FOR PRODUCING 1,2-DICHLORETHANE BY MEANS OF DIRECT CHLORINATION
PROCEDE ET DISPOSITIF DE PRODUCTION DE 1,2-DICHLORETHANE PAR CHLORATION DIRECTE

(30) Priorität: 17.06.2004 DE 102004029147
(43) Veröffentlichungstag der Anmeldung: 28.02.2007
(73) Patentinhaber: Uhde GmbH, 44141 Dortmund (DE); Vinnolit GmbH & Co. KG, 84504 Burgkirchen (DE)
(72) Erfinder: BENJE, Michael, 64289 Darmstadt (DE); HAFENSCHER, Harald, 65843 Sulzbach (DE)
(86) Internationale Anmeldenummer: PCT/EP2005/006437
(87) Internationale Veröffentlichungsnummer: WO 2005/123635

(56) Entgegenhaltungen:
- EP-A- 0 026 349
- WO-A-03/074167
- JP-A- 6 157 365
- US-A- 4 783 564
- US-A- 5 177 233
- US-A1- 2004 116 625

## Beschreibung

Die Erfindung richtet sich auf ein Verfahren und eine Vorrichtung zur Herstellung von 1,2-Dichlorethan, im folgenden als EDC bezeichnet, welches überwiegend als Zwischenprodukt der Herstellung von monomerem Vinylchlorid, im folgenden als VCM bezeichnet, dient, woraus letztlich Polyvinylchlorid, PVC, hergestellt wird. Bei der Umsetzung von EDC zu VCM entsteht Chlorwasserstoff HCI. EDC wird daher bevorzugt aus Ethylen C₂H₄ und Chlor Cl₂ derart hergestellt, dass hinsichtlich des bei den Umsetzungen erzeugten und verbrauchten Chlorwasserstoffes HCl eine ausgewogene Bilanz entsprechend den folgenden Reaktionsgleichungen erreicht wird:

Cl₂ + C₂H₄ → C₂H₄Cl₂ (Rein-EDC) + 218 kJ/Mol (1)

C₂H₄Cl₂ (Spalt-EDC) → C₂H₃Cl (VCM) + HCI - 71 kJ/Mol (2)

C₂H₄ + 2 HCI + ½ O₂ → C₂H₄Cl₂ (Roh-EDC) + H₂O + 238 kJ/Mol (3)

Das Verfahren zur Herstellung von VCM mit ausgewogener HCI-Bilanz, im folgenden kurz "ausgewogenes VCM-Verfahren" genannt, besitzt:
- eine Direktchlorierung, in der aus Ethylen C₂H₄ und Chlor Cl₂ der eine Teil des benötigten EDC in Gegenwart eines Homogenkatalysators erzeugt wird und als sogenanntes Rein-EDC abgegeben wird;
- eine Oxichlorierung, in der aus Ethylen C₂H₄, Chlorwasserstoff HCl und Sauerstoff O₂ der andere Teil des EDC erzeugt wird und als sogenanntes Roh-EDC abgegeben wird;
- eine fraktionierende EDC-Reinigung, in der das Roh-EDC zusammen mit dem aus der VCM-Fraktionierung rezirkulierten Rück-EDC und optional zusammen mit dem Rein-EDC von den in der Oxichlorierung und von den in der EDC-Pyrolyse gebildeten Nebenprodukten befreit wird, um ein für den Einsatz in der EDC-Pyrolyse geeignetes, sogenanntes Feed-EDC zu gewinnen, wahlweise kann auch das aus der Direktchlorierung stammende Rein-EDC in der Hochsiederkolonne der EDC-Destillation mitdestilliert werden;
- eine EDC-Pyrolyse, in der das Feed-EDC thermisch gespalten wird; das Spaltgas genannte Reaktoraustrittsgemisch enthält VCM, Chlorwasserstoff HCI und nichtumgesetztes EDC sowie Nebenprodukte;
- eine VCM-Fraktionierung, in der das als Produkt gewünschte Rein-VCM aus dem Spaltgas abgetrennt und die anderen wesentlichen Spaltgasbestandteile Chlorwasserstoff HCI und nichtumgesetztes EDC als Wertstoffe gesondert zurückgewonnen und als wiederverwertbarer Einsatz als Rück-HCl bzw. Rück-EDC im ausgewogenen VCM-Verfahren rezirkuliert werden.

Als Reaktionsmedium in der Direktchlorierung dient bei den meisten im industriellen Maßstab angewandten Verfahren ein umlaufender Strom des Reaktionsprodukts EDC. Dieser kann in einem Schlaufenreaktor mit äußerem oder innerem Umlauf erzeugt werden. Weiterhin kann der Umlaufstrom durch Zwangsumlauf oder Naturumlauf erzeugt werden. Als Katalysator wird vor allem Eisen-III-Chlorid verwendet; zusätzlich kann Natriumchlorid, das in der Lage ist, die Bildung von Hochsiedern zu vermindern, als Additiv verwendet werden.

Der Stand der Technik zur Direktchlorierung wird z.B. in der DE 199 10 964 A1 beschrieben. In dem in DE 199 10 964 A1 beschriebenen Verfahren sollen Nebenreaktionen, vor allem die Weiterchlorierung des EDC zum 1,1,2-Trichlorethan, dadurch unterdrückt werden, dass die Chlorierungsreaktion weitgehend in homogener, flüssiger Phase abläuft. Das in EDC schwerer als Chlor lösliche Ethylen wird im Hauptstrom des umlaufenden Reaktionsmediums EDC in einer Gleichstromblasensäule vollständig aufgelöst. Das in EDC leichter als Ethylen lösliche Chlor wird in einem unterkühlten EDC-Teilstrom aufgelöst und die so erhaltene Lösung von Chlor in EDC dem umlaufenden Hauptstrom, in dem das Ethylen bereits gelöst vorliegt, zugegeben.

Die Reaktion (1) wird üblicherweise mit einem geringen Ethylenüberschuss betrieben, um Korrosionsprobleme im Reaktionssystem, erhöhte Nebenproduktbildung sowie Probleme, die mit der Behandlung chlorhaltiger Abgasströme verbunden sind, auf jeden Fall zu vermeiden. Chlor und Ethylen werden dem Reaktor über eine Verhältnisregelung zugeführt; als Führungsgrösse dient der Ethylengehalt des Reaktoraustrittsstroms. Hierbei ist man aus wirtschaftlichen Gründen stets bestrebt, den Ethylenüberschuss am Reaktoraustritt so niedrig wie möglich zu halten, um zu große Ethylenverluste zu vermeiden.

Es hat sich ferner herausgestellt, dass die Reaktion (1) besonders dann ohne große Nebenproduktbildung abläuft, wenn sie vollständig als Flüssigphasenreaktion betrieben wird, wie es auch in der WO 03/070673 A1 beschrieben wird. Hierzu ist es erforderlich, dass das Ethylen im Reaktionsrohr noch vor der Zugabe von gelöstem Chlor vollständig aufgelöst ist. Die vom Gasverteiler anfänglich erzeugten kleinen Gasblasen wachsen längs dieser Strecke durch Kolaeszenz an und erreichen schließlich eine durch Kolaeszenz - und Zerfallsvorgänge bedingte, konstante Gleichgewichtsgröße. Hier handelt es sich um einen Effekt, der den Stoffaustausch ungünstig beeinflusst, da sich durch die Vergrößerung des Blasendurchmessers bei einem bestimmten Gasgesamtvolumen die für den Stoffaustausch zu Verfügung stehende Oberfläche verkleinert.

Die Reaktion (1) in der anschließenden, weitgehend homogenen Reaktionszone verläuft kinetisch nach einem Geschwindigkeitsgesetz 2. Ordnung. Liegen beide Reaktionspartner anfänglich im stöchiometrischen Verhältnis vor oder liegt ein leichter Überschuss eines der Reaktionspartner vor, so verläuft die Reaktion langsamer, als wenn einer der Reaktionspartner, z.B. das Ethylen in Reaktion (1) anfänglich in größerem Überschuss vorliegt. Für einen Reaktor der hier beschriebenen Art bedeutet dies, dass die Reaktion nach einer kürzeren Laufstrecke im umlaufenden Strom des Reaktionsmediums abgeschlossen ist, als es bei einem geringeren anfänglichen Ethylenüberschuss der Fall wäre.

Die Überlagerung der Effekte bei der Auflösung des Ethylens, bei der Reaktion selbst und dem Einsetzen des Siedens definieren beim herkömmlichen Stand der Technik die Dimensionierung des Siedereaktors und erschweren eine nachträgliche Kapazitätserhöhung erheblich.

Die Aufgabe der Erfindung besteht daher darin, ein wirtschaftliches Verfahren sowie eine dazugehörige Vorrichtung zur Verfügung zu stellen, welches eine Kapazitätsvergrößerung ermöglicht, ohne die äußeren Reaktorabmessungen zu vergrößern und gleichzeitig EDC hoher Reinheit erzeugt.

Diese Aufgabe wird durch das Verfahren nach Patentanspruch 1 gelöst. Die Erfindung löst die Aufgabe, indem
- eine Vielzahl von mindestens 3 Zugabeabschnitten in dem Schenkel der Schlaufe angeordnet sind, in dem die Flüssigkeit aufsteigt, und
- jeder dieser Zugabeabschnitte aus einer stromaufwärts gelegenen Einspeisung von gelöstem oder gasförmigem Ethylen und einer stromabwärts gelegenen Einspeisung von gelöstem Chlor besteht und weiterhin eine statische Mischvorrichtung aufweisen kann.
Die Einspeisung wird üblicherweise als Eindüsung ausgeführt, kann aber auch anders ausgeprägt sein.

Durch die Installation mehrerer, durch die oben beschriebenen Zugabeabschnitte definierter Reaktionsstrecken im Steigrohr der Reaktorschlaufe ergibt sich folgender Vorteil: Durch die Hintereinanderschaltung können alle Reaktionsstrecken ausser der letzten mit einem hohen Ethylenüberschuss betrieben werden, was zu einer erheblichen Verkürzung der Reaktionsstrecken führt und damit die Möglichkeit eröffnet, mehrere Reaktionsstrecken in einem Reaktionsrohr wirtschaftlicher Baugrösse unterzubringen. So können bei einer bestimmten Reaktorgröße sehr hohe Produktionskapazitäten realisiert werden. Das nicht umgesetzte Ethylen der jeweils vorgeschalteten Reaktionsstrecke wird in gelöster Form in die nächste Löse- bzw. Reaktionsstrecke transportiert. Das Ethylen-Chlorverhältnis aller Reaktionsstrecken außer der letzten, vor dem Reaktoraustritt angeordneten, wird vorzugsweise fest eingestellt. Das Ethylen-Chlorverhältnis der letzten Reaktionsstrecke dagegen wird in herkömmlicher Art geregelt, wobei als Führungsgröße der Ethylengehalt des den Reaktor verlassenden Gasstroms dient.

Dabei ergibt sich zusätzlich der überraschende Vorteil, dass die Ethylenverluste bezogen auf die Menge an produziertem EDC umso geringer werden, je mehr Reaktionsstrecken installiert werden bzw. je mehr EDC in einer Reaktorschlaufe der hier beschriebenen Art produziert wird. Dies liegt darin begründet, dass die Ethylenverluste des Gesamtsystems nur denjenigen der letzten Reaktionsstrecke entsprechen, während die vorgeschalteten Reaktionsstrecken verlustfrei betrieben werden. Da die Reaktion analog der in der WO 03/070673 A1 beschriebenen Verfahrensweise weiterhin in der flüssigen Phase stattfindet, bleiben die Vorteile dieses Systems, insbesondere die sehr geringe Nebenproduktbildung auch bei relativ hohen Reaktionstemperaturen voll erhalten. Eine zusätzliche Verkürzung der Reaktionsstrecken lässt sich erreichen, wenn die oben beschriebene Serienschaltung von Reaktionsstrecken mit einer Unterstützung des Vorgangs der Ethylenauflösung bzw. der Reaktion durch den Einsatz statischer Mischelemente kombiniert wird.

Dadurch, dass mehrere Ethylen-Zugabestellen im Steigrohr der Reaktorschlaufe installiert werden, erhöht sich der mittlere Gasgehalt im Steigrohr und damit auch die treibende Kraft, die für einen Naturumlauf stets erforderlich ist. Daher wird es möglich, statische Mischer in die jeweiligen Reaktionsstrecken zu integrieren, deren Einsatz zwar aus der EP 907 626 A1 im Grunde bekannt ist, die aber aufgrund ihres Druckverlustes in anderen Verfahren mit einem energieintensiven, die Wirtschaftlichkeit des Verfahrens verschlechternden Umpump betrieben werden müssen bzw. bei Installation in einer Reaktorschlaufe mit Naturumlauf deren Regelverhalten, insbesondere das Teillastverhalten nachteilig beeinflussen.

In einer weiteren Ausgestaltung der Erfindung wird vorgesehen, dass in allen Ethylen-Zugabestellen, mit Ausnahme des stromabwärts letzten Zugabeabschnittes, eine jeweils stark überstöchiometrische Ethylenmenge zugeführt wird. Als besonders wirtschaftlich hat sich die Zugabe einer um 10 bis 20 % überstöchiometrischen Ethylenmenge herausgestellt. Durch die vermehrte Zugabe vergrößert sich zwar die jeweilige Lösestrecke entsprechend, dafür verläuft aber die Reaktion insgesamt schneller, was summarisch zu einer Bauhöheneinsparung führt und es ermöglicht, mehr Zugabestellen unterzubringen und den Reaktionsumsatz zu vergrößern.

Insgesamt ist es auf diese Weise möglich, mit einem Siedereaktor herkömmlicher Baugröße durch Umbau in der erfindungsgemäßen Weise mindestens den doppelten Umsatz nach Reaktion (1) zu erreichen. Der große Vorteil der Erfindung besteht daher in der unkomplizierten Nachrüstbarkeit bei Kapazitätserhöhungen in bestehenden Anlagen. Selbstverständlich ist es auch wirtschaftlich besonders bei großen Anlagen sinnvoll, Siedereaktoren schon bei der ursprünglichen Planung mit den erfindungsgemäßen Aufgabevorrichtungen auszustatten.

Der erfindungsgemäße Siedereaktor zeichnet sich auch durch ein ausgezeichnetes Teillastverhalten aus, da einzelne Zugabestellen auch während der Fahrt außer Betrieb genommen werden können, was ein weiterer Vorteil der Erfindung ist. Zweckmäßiger Weise schaltet man zunächst die weiter oben gelegenen Zugabestellen ab und senkt den Druck, so dass das erzeugte EDC früher siedet. Hierdurch kann auch bei kleineren Umsätzen der Druckverlust ausgeglichen werden, den statische Mischer verursachen, sofern sie vorgesehen werden.

Die Erfindung umfasst auch die Vorrichtung zur Durchführung des Verfahrens mit einem Siedereaktor, welcher aus einem Ausgasgefäß, einer im Naturumlauf betriebenen Reaktionsschlaufe sowie Abzugsvorrichtungen für erzeugtes EDC besteht, und je mindestens 2 Zugabevorrichtungen für gelöstes Chlor und für Ethylen. Die Vorrichtung kann optional auch statische Mischvorrichtungen enthalten.

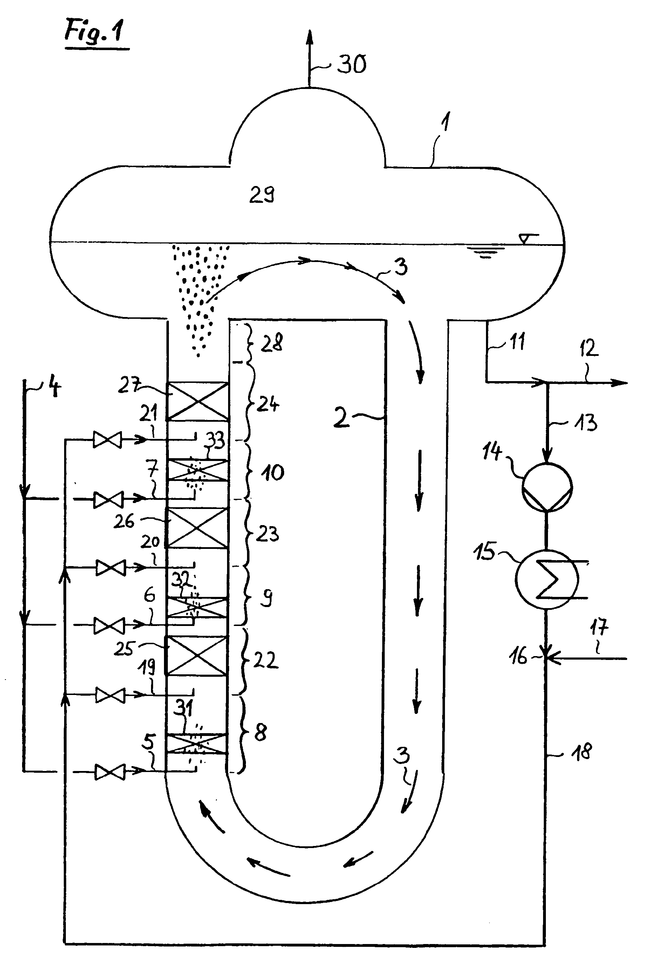

Die Erfindung wird im folgenden anhand eines Beispiels näher erläutert. Fig. 1 zeigt einen Direktchlorierungsreaktor, der aus einem Ausgasgefäß 1 und einer Schlaufe 2 besteht, in der flüssiges EDC 3, angedeutet durch Pfeile, umläuft, und in welcher die Reaktion (1) durchgeführt wird, sowie die erfindungsgemäßen Zugabestellen für Chlor und Ethylen.

Gasförmiges, unter Druck stehendes Ethylen 4 wird über Regelventile auf die Ethylen-Zugabestellen 5, 6 und 7 verteilt. Die Ethylen-Zugabestellen werden so ausgeführt, dass feine Blasen, in Fig. 1 durch Punkte symbolisch angedeutet, entstehen, die sich in den Auflösestrecken 8, 9 und 10 schnell auflösen können. In die Auflösestrecken 8, 9 und 10 können optional die statischen Mischer 31, 32 und 33 integriert werden.

Das einzubringende Chlor wird zunächst in EDC gelöst. Hierzu wird ein Teilstrom 11 des erzeugten EDC aus dem Ausgasgefäß 1 abgezogen und in einen EDC-Strom 12, der auch ein diskontinuierlicher Ausschleusestrom sein kann, und in einen Lösungsstrom 13 aufgeteilt. Der Lösungsstrom 13 wird anschließend von der Pumpe 14 in den Kühler 15 und nachfolgend zur Chloreinmischung 16 gefördert, wo das gasförmige Chlor 17 zugeführt wird. Die Chlorlösung 18 wird geregelt auf die Chlor-Zugabestellen 19, 20 und 21 verteilt und mit Düsen in die Schlaufe 2 eingedüst, wobei eine möglichst große Verwirbelung erzeugt wird.

Oberhalb der Chlor-Zugabestellen 19, 20 und 21 schließen sich die Reaktionsstrecken 22, 23 und 24 an, in denen die Reaktion 1 in der reinen Flüssigphase stattfindet. Um die Vermischung zu verbessern, sind in die Reaktionsstrecken 22, 23 und 24 die statischen Mischer 25, 26 und 27 integriert.

Im oberen Bereich der Schlaufe 2 schließt sich an die Reaktionsstrecke 24 der Siedebereich 28 an, was durch Blasenbildung in Fig. 1 angedeutet ist. Das dampfförmige EDC gast in den Dampfraum 29 des Ausgasgefäßes 1 aus und wird als dampfförmiges EDC-Produkt 30 abgeführt.

Die Erfindung wird nachfolgend anhand eines gerechneten Beispiels näher erläutert. In der Reaktorschlaufe 2 laufen 8000 t/h flüssiges EDC 3 mit einer Temperatur von 90 bis 135°C um. Über die Zugabestelle 5 wird eine Menge von 8892 kg/h gasförmigem Ethylen, entsprechend 317 kmol/h eingespeist, das sich längs der Laufstrecke 8 in dem umlaufenden EDC 3 löst.

Aus dem Ausgasgefäß 1 wird eine Menge von 1140 t/h flüssigem EDC (Strom 13) entnommen, mit der Pumpe 14 durch den Wärmetauscher 15 gefördert und dort auf eine Temperatur von 30 bis 60 °C abgekühlt. An der Chloreinmischung 16 wird dem abgekühlten EDC-Strom eine Chlormenge von 61260 kg/h, entsprechend 864 kmol/h, gasförmigem oder flüssigem Chlor zugemischt. Die so erhaltene Chlorlösung 18 wird in drei gleiche Teilströme aufgeteilt, die aus jeweils 380 t/h EDC und 20420 kg/h Chlor, entsprechend 288 kmol/h Chlor bestehen. Diese drei Teilströme werden über die Zugabestellen 19, 20 und 21 in die Reaktorschlaufe 2 eingespeist.

Alternativ kann auch so verfahren werden (in Fig. 1 nicht dargestellt), dass der abgekühlte EDC-Strom von 1140 t/h EDC zunächst in drei gleiche Teilströme von je 380 t/h EDC aufgeteilt wird und jedem dieser drei EDC-Teilströme über eine separate Chloreinmischung eine Menge von 20420 kg/h Chlor, entsprechend 288 kmol/h gasförmigem oder flüssigem Chlor zugemischt wird. Auch hier werden drei Teilströme einer Lösung von Chlor in EDC erhalten, die aus jeweils 380 t/h EDC und 20420 kg/h Chlor bestehen und über die Zugabestellen 19, 20 und 21 in die Reaktorschlaufe 2 eingespeist werden.

Der von unten nach oben im Steigrohr der Reaktorschlaufe gesehen erste Ethylenstrom 5, der sich längs der Strecke 8 im umlaufenden EDC-Strom 3 gelöst hat, wird mit dem Teilstrom 19 der Lösung von Chlor in EDC gemischt. Die Reaktionspartner Chlor und Ethylen reagieren in der flüssigen Phase längs der Reaktionsstrecke 22 unter Bildung von EDC. Da das gelöste Ethylen in einem molaren Überschuss von 10 % vorliegt, erfolgt die Abreaktion des gelösten Chlors deutlich schneller, als wenn die Reaktionspartner äquimolar vorlägen oder auch als wenn das Ethylen nur in einem geringen Überschuss vorläge. Die Reaktionswärme führt zu einer Erwärmung des umlaufenden EDC 3. Da die zugemischte Chlorlösung eine deutlich niedrigere Temperatur hat als umlaufendes EDC 3, wird ein Teil der Reaktionswärme durch die Erwärmung der Chlorlösung auf das Temperaturniveau des Reaktorinhalts abgeführt. Hierdurch, sowie durch die geeignete Einstellung des Reaktordrucks, lässt sich ein Sieden in den unteren Reaktionszonen und damit eine verstärkte Bildung von Nebenprodukten unterdrücken.

Nach Abreaktion des gelösten Chlors enthält das umlaufende EDC 3, das nun in die von unten im Steigrohr der Reaktorschlaufe gezählt zweite Ethylen-Lösestrecke 9 eintritt, bereits gelöstes Ethylen. In die Ethylen-Lösestrecke 9 wird mit dem EDC 3 eine Ethylenmenge von 814 kg/h, entsprechend 29 kmol/h gelöstem Ethylen, transportiert. Nun wird über die Ethylen-Zugabestelle 6 eine Menge von 8078 kg/h, entsprechend 288 kmol/h gasförmiges Ethylen eingespeist, das sich längs der Ethylen-Lösestrecke 9 im umlaufenden EDC 3 auflöst. Nach Durchlaufen der Ethylen-Lösestrecke 9 liegen wieder 8892 kg/h entsprechend 317 kmol/h gelöstes Ethylen im EDC 3 vor.

In analoger Weise erfolgt nun die Zugabe des Teilstroms 20 von in EDC gelöstem Chlor sowie die Reaktion von Chlor und Ethylen unter Bildung von EDC längs der Reaktionsstrecke 23. Auch hier wird durch die Kühlwirkung des gekühlten Chlor-Lösungsstroms sowie durch geeignete Einstellung des Reaktordrucks das Sieden des Reaktionsgemischs unterdrückt. Durch den molaren Ethylenüberschuss von 10 % erfolgt auch hier eine rasche Abreaktion des Chlors.

Da Ethylenverluste ebenso wie ein zu hoher Anteil an gelöstem Ethylen im produzierten EDC wirtschaftlich nachteilig bzw. in nachgeschalteten Prozessschritten unerwünscht sind, wird die im Steigrohr der Reaktorschlaufe letzte, am höchsten angeordnete Reaktionsstrecke mit einem nur geringen Ethylenüberschuss betrieben.

Ebenso wie nach der ersten Reaktionsstrecke wird eine gelöste Ethylenmenge von 814 kg/h, entsprechend 29 kmol/h, in die Ethylen-Lösestrecke 10 transportiert. Über die Ethylen-Einspeisung 7 wird eine Menge von 7289 kg/h, entsprechend 260 kmol/h, gasförmiges Ethylen eingespeist, das sich längs der Ethylen-Lösestrecke 10 im umlaufenden EDC löst.

Danach wird der Teilstrom 21 von in EDC gelöstem Chlor zugegeben. Nun liegt das Ethylen lediglich in einem molaren Überschuss von 0,35 % vor, wodurch sich die Reaktionsstrecke 24 gegenüber den Reaktionsstrecken 22 und 23 deutlich verlängert. Nach Durchlaufen der Reaktionsstrecke 24 nimmt der hydrostatische Druck so weit ab, dass das Reaktionsgemisch zu sieden beginnt und das Reaktionsprodukt gasförmig aus dem Ausgasgefäß 1 abgezogen werden kann.

### Bezugszeichenliste

- 1: Ausgasgefäß
- 2: Schlaufe
- 3: flüssiges EDC
- 4: Ethylen
- 5: Ethylen-Zugabestelle
- 6: Ethylen-Zugabestelle
- 7: Ethylen-Zugabestelle
- 8: Auflösestrecke
- 9: Auflösestrecke
- 10: Auflösestrecke
- 11: Teilstrom
- 12: EDC-Strom
- 13: Lösungsstrom
- 14: Pumpe
- 15: Kühler
- 16: Chloreinmischung
- 17: gasförmiges Chlor
- 18: Chlorlösung
- 19: Chlor-Zugabestelle
- 20: Chlor-Zugabestelle
- 21: Chlor-Zugabestelle
- 22: Reaktionsstrecke
- 23: Reaktionsstrecke
- 24: Reaktionsstrecke
- 25: statischer Mischer
- 26: statischer Mischer
- 27: statischer Mischer
- 28: Siedebereich
- 29: Dampfraum
- 30: dampfförmiges EDC-Produkt
- 31: statischer Mischer
- 32: statischer Mischer
- 33: statischer Mischer

## Patentansprüche

1. Verfahren zur Herstellung von 1,2-Dichlorethan hoher Reinheit aus gelöstem Chlor und gelöstem Ethylen, welche miteinander in Kontakt gebracht werden, unter Einsatz eines im Umlauf geführten flüssigen Reaktionsmediums, welches im Wesentlichen aus 1,2-Dichlorethan und einem Katalysator besteht und mindestens eine vertikal angeordnete, als Schlaufe ausgebildete Reaktionsstrecke durchläuft, wobei die beiden Schenkel der Schlaufe mit einem oberseitig angeordneten Ausgasbehälter verbunden sind, von dem aus das Reaktionsprodukt entweder gasförmig oder flüssig oder sowohl gasförmig als auch flüssig ausgeschleust wird, **dadurch gekennzeichnet, dass**
• eine Vielzahl von mindestens 3 Zugabeabschnitten in dem Schenkel der Schlaufe angeordnet sind, in dem die Flüssigkeit aufsteigt, und
• jeder dieser Zugabeabschnitte aus einer stromaufwärts gelegenen Einspeisung von gelöstem oder gasförmigem Ethylen und einer stromabwärts gelegenen Einspeisung von gelöstem Chlor besteht und weiterhin statische Mischvorrichtungen aufweisen kann.

2. Verfahren nach Anspruch 1, **dadurch gekennzeichnet, dass** in allen Ethylen-Zugabestellen, mit Ausnahme des stromabwärts letzten Zugabeabschnittes, eine jeweils stark überstöchiometrische Ethylenmenge zugeführt wird.

3. Schlaufenreaktor zur Durchführung des Verfahrens gemäß Anspruch 1,
• aufweisend eine vertikal angeordnete, als Schlaufe ausgebildete Reaktionsstrecke,
• wobei die beiden Schenkel der Schlaufe mit einem oberseitig angeordneten Ausgasbehälter verbunden sind, von dem aus das Reaktionsprodukt entweder gasförmig oder flüssig oder sowohl gasförmig als auch flüssig ausgeschleust werden kann,
• aufweisend mindestens 3 Zugabeabschnitte, die in dem Schenkel der Schlaufe angeordnet sind, in dem die Flüssigkeit aufsteigt,
• wobei jeder dieser Zugabeabschnitte aus einer stromaufwärts gelegenen Einspeisung von gelöstem oder gasförmigem Ethylen und einer stromabwärts gelegenen Einspeisung von gelöstem Chlor besteht,
• und weiterhin statische Mischvorrichtungen aufweisen kann.

## Claims

1. Process for the production of 1,2-dichloroethane of high purity from solute chlorine and solute ethylene, which are brought into contact with each other in a circulated liquid reaction fluid, which mainly consists of 1,2-dichloroethane and a catalyst and flows through at least one vertically arranged loop-type reaction section, both legs of the loop being connected to a degassing vessel arranged at the top from which the reaction product is withdrawn either in the gaseous or in the liquid phase or in both the gaseous and the liquid phase,
**characterized in that**
• a plurality of at least 3 admixing sections is arranged in the leg of the loop in which the liquid rises,
• each of these admixing sections has one feed point for solute or gaseous ethylene, which is arranged upstream, and one feed point for solute chlorine, which is located downstream, and may have static mixers.

2. Process according to claim 1, **characterised in that** a highly hyperstoichiometric amount of ethylene is supplied to all ethylene feed points except that of the downstream last admixing section.

3. Loop reactor for running the process as described in claim 1,
• provided with a vertically arranged loop-type reaction section,
• both legs of the loop being connected to a degassing vessel arranged at the top from which the reaction product can be withdrawn either in the gaseous or in the liquid phase or in both the gaseous and the liquid phase,
• provided with at least 3 admixing sections arranged in the leg of the loop in which the liquid rises,
• each of these admixing sections having one feed point for solute or gaseous ethylene, which is arranged upstream, and one feed point for solute chlorine, which is located downstream,
• and possibly being provided with static mixers.

## Revendications

1. Procédé pour la production de 1,2-dichloroéthane, de haute pureté, à partir de chlore dissous et d'éthylène dissous, qui sont mis au contact entr'eux, en présence d'un flux circulé d'un agent de réaction liquide, qui consiste principalement en 1,2-dichloroéthane et un liquide catalytique et qui parcourt au minimum une unité verticale de réaction dessinée comme une boucle, dont les deux sections de côté sont branchées sur un récipient de dégazage disposé au-dessus, le dit récipient de dégazage étant utilisé pour l'évacuation du produit de réaction sous forme gazeuse ou liquide ou bien sous forme gazeuse et liquide,
**caractérisé en ce que**:
• une multitude d'au moins 3 secteurs d'addition est disposée dans les deux sections de côté de la boucle, dans laquelle le liquide monte vers le haut, et que
• chaque secteur d'addition consiste en un point d'amenée d'éthylène dissous ou gazeux, disposé en amont, et en un point d'amenée de chlore dissous, disposé en aval et, de plus, en des dispositifs mélangeurs statiques.

2. Procédé selon la revendication no. 1, **caractérisé en ce qu'**une quantité d'éthylène fortement hyperstoechiométrique est envoyée à chaque point d'amenée, sauf au dernier secteur d'addition disposé en aval.

3. Réacteur à boucle pour la réalisation du procédé, selon la revendication no. 1, mettant en oeuvre l'équipement spécifié ci-après:
• Une unité de réaction, en position verticale, et dessinée comme une boucle ;
• les deux sections de côté de la boucle sont branchées sur un récipient de dégazage disposé au-dessus et utilisé pour l'évacuation du produit de réaction sous forme gazeuse ou liquide ou bien sous forme gazeuse et liquide;
• l'unité est munie d'un minimum de trois secteurs d'addition disposés dans la section de côté de la boucle, dans laquelle le liquide monte vers le haut ;
• chaque secteur d'addition consiste en un point d'amenée d'éthylène dissous ou gazeux, disposé en amont, et en un point d'amenée de chlore dissous, disposé en aval ;
• et, de plus, l'unité peut être dotée des dits dispositifs mélangeurs statiques.
